# EUROPÄISCHE PATENTANMELDUNG

(11) **EP 2 015 210 A2**
(43) Veröffentlichungstag der Anmeldung: **14.01.2009**
(21) Anmeldenummer: 08157533.4
(22) Anmeldetag: 04.06.2008
(51) Int. Cl.: G06F 19/00

(54) **Aktives medizinisches Implantat**

(30) Priorität: 06.07.2007 DE 102007031713
(71) Anmelder: BIOTRONIK CRM Patent AG, 6341 Baar (CH)
(72) Erfinder: Kukla, Volker, 12157 Berlin (DE); Reinke, Joachim, 10439 Berlin (DE); Depping, Nicole, 10999 Berlin (DE)
(74) Vertreter: Lindner-Vogt, Karin L.

(57) **Zusammenfassung**

Die Erfindung besteht in einer Anordnung für die Fernprogrammierung eines implanticrbaren medizinischen Gerätes wie ein Herzschrittmacher, Defibrillator oder dergleichen. Komponenten der Anordnung sind ein programmierbares persönliches Gerät (10; 20) und ein Service Center (30). Das Service Center (30) weist eine Programmierüberwachungseinheit (40) auf, die ausgebildet ist, einen vom Zeitpunkt des Absendens des Programmierauftrags abhängigen Programmierschlusszeitpunkt zu bestimmen und den Programmierauftrag zu stornieren oder zu löschen, falls das Service Center bis zum Programmierschlusszeitpunkt keine eine erfolgreiche Ausführung des Programmierauftrags bestätigende Programmierbestätigung seitens des programmierbaren persönlichen Gerätes empfangen hat, und das programmierbare medizinische Implantat ist dazu ausgebildet, nach Empfang eines Programmierauftrags oder nach erfolgreicher Weiterleitung eines Programmierauftrags eine Programmierbestätigung an das Service Center abzusenden.

## Beschreibung

Die Erfindung betrifft eine Anordnung und ein Verfahren für die Fernprogrammierung eines programmierbaren persönlichen medizinischen Gerätes, insbesondere eines implantierbaren medizinischen Gerätes, wie beispielsweise einem Herzschrittmacher, einem Defibrillator, einem Neurostimulator oder dergleichen. Die Anordnung umfasst wenigstens die Komponenten programmierbares persönliches Gerät und ein zentrales Servicecenter. Das persönliche medizinische Gerät besitzt eine Datenkommunikationsschnittstelle, die so ausgebildet ist, dass das persönliche medizinische Gerät wenigstens mittelbar und bidirektional Daten mit dem Servicecenter austauschen kann. Das Servicecenter besitzt ebenfalls eine Datenkommunikationsschnittstelle, um eine wenigstens mittelbare Verbindung zu dem persönlichen medizinischen Gerät herzustellen und über diese bidirektional Daten auszutauschen. Außerdem besitzt das Servicecenter eine Benutzerschnittstelle, die so ausgebildet ist, dass mit ihrer Hilfe Programmieraufträge für das programmierbare persönliche Gerät zusammengestellt werden können und das Absenden eines zusammengestellten Programmierauftrags ausgelöst werden kann.

Derartige Anordnungen sind insbesondere im Zusammenhang mit Herzschrittmachern oder Defibrillatoren inzwischen hinlänglich bekannt und erlauben es beispielsweise, seitens eines Herzschrittmachers oder Defibrillators gewonnene Daten oder deren Betriebsdaten zu einem zentralen Servicecenter zu übertragen, um diese Daten dort auszuwerten und über eine entsprechende Benutzerschnittstelle einem betreuenden Arzt zur Verfügung zu stellen. Einige Funktionen solcher Implantate sind durch Software oder Firmware gesteuert und daher programmierbar. Dabei kommt es immer wieder vor, dass nach anfänglicher Umprogrammierung des Implantats kurz vor, während oder kurz nach der Implantation weitere Programmierungen oder Umprogrammierungen wünschenswert sind, um das Implantat besser auf möglicherweise inzwischen veränderte Gesundheitszustände eines Patienten einstellen zu können oder die Leistungsfähigkeit des Implantats anderweitig zu erhöhen. Ein derartiges Programmieren oder Umprogrammieren kann zum einen dadurch geschehen, dass ein Arzt zu einem Implantat mit Hilfe eines Programmiergerätes eine kurzreichweitige, drahtlose Datenverbindung herstellt und das Implantat im Angesicht des Patienten programmiert.

Eine Programmierung oder Umprogrammierung des Implantates kann jedoch auch über das zentrale Servicecenter geschehen. Dazu besteht üblicherweise eine Datenverbindung zwischen dem Servicecenter und einem Patientengerät, welches sich üblicherweise in der Nähe eines Patienten befindet und eine bidirektionale Datenverbindung zwischen dem Implantat und dem Patientengerät herstellen kann. Die Verbindung zwischen Servicecenter und Patientengerät kann dabei drahtlos oder drahtgebunden, beispielsweise über das Telefonnetz, ausgebildet sein.

Eine Anordnung zur Fernprogrammierung von Implantaten ist beispielsweise in US 6,442,432 beschrieben.

Der vorliegenden Erfindung liegt die Aufgabe zugrunde, grundsätzlich bekannte Anordnungen und Verfahren zur Fernprogrammierung von persönlichen medizinischen Geräten, insbesondere von Implantaten, wie Herzschrittmachern, Defibrillatoren oder Kardiovertem, noch sicherer zu machen.

Erfindungsgemäß wird diese Aufgabe durch eine Anordnung der eingangs genannten Art gelöst, bei der das Servicecenter eine Programmierüberwachungseinheit aufweist, die ausgebildet ist, einen vom Zeitpunkt des Absendens des Programmierauftrags abhängigen Programmierschlusszeitpunkt zu bestimmen und einen bereits abgesandten Programmierauftrag dann zu löschen, wenn das Servicecenter bis zum Programmierschlusszeitpunkt keine eine erfolgreiche Ausführung des Programmierauftrags bestätigende Programmierbestätigung seitens des programmierbaren persönlichen medizinischen Gerätes empfangen hat.

Mit einer derartigen Programmierüberwachungseinheit kann sichergestellt werden, dass ein einmal abgesandter Programmierauftrag nicht dann erst zur Ausführung kommt, wenn er möglicherweise bereits überholt ist. Die Erfindung trägt der Erkenntnis Rechnung, dass für eine Übertragung eines Programmierauftrages nicht immer eine hundertprozentige Zuverlässigkeit sichergestellt werden kann und dass eine derartige Übertragung auch mehrere Tage oder Wochen in Anspruch nehmen kann. Damit dieses Problem nicht dazu führt, dass ein längst veralteter Programmierauftrag dann zu einer Programmierung des Implantates führt, wenn diese Programmierung gar nicht mehr gewollt ist, stellt das Servicecenter sicher, dass der Programmierauftrag gelöscht wird, sobald der Programmierschlusszeitpunkt überschritten ist und die Programmierung bis dahin nicht erfolgt ist.

Hinter dieser Erfindung steckt die weitere Erkenntnis, dass die Programmierung möglicherweise deswegen nicht innerhalb der gesetzten Zeit ausgeführt wird, weil die Übertragung des Programmierauftrags über möglicherweise mehrere Geräte nicht zustande kommt. Häufig ist dabei die Übertragungskette an irgendeinem Punkt unterbrochen. Ein abgesandter Programmierauftrag wird dabei bis zum letzten Gerät vor der Unterbrechung weitergeleitet und bleibt dort gewissermaßen stecken. In einem solchen Szenario ist es sehr wahrscheinlich, dass ein späterer Programmierlöschauftrag seitens des Servicecenters den zuvor abgesandten Programmierauftrag einholt, und zwar an dem Gerät, an dem der Programmierauftrag infolge der Unterbrechung der Übertragungskette hängen geblieben ist.

Entsprechend ist das Servicecenter vorzugsweise dazu ausgebildet, nach Verstreichen des Programmierschlusszeitpunkts einen Programmierlöschauftrag auszusenden, falls bis zum Programmierschlusszeitpunkt keine Programmierbestätigung eingetroffen ist, die die erfolgreiche Ausführung der Programmierung des Implantats oder wenigstens die erfolgreiche Übertragung an ein jeweiliges, dem Implantat unmittelbar zugeordnetes persönliches medizinisches Gerät eingetroffen ist.

Vorzugsweise umfasst die Anordnung neben dem Servicecenter und dem programmierbaren persönlichen medizinischen Gerät auch weitere Geräte, insbesondere ein Implantat. Das Implantat besitzt eine Schnittstelle für die drahtlose, bidirektionale Datenübertragung zwischen dem persönlichen medizinischen Gerät und dem Implantat. Eine hiermit kompatible Datenschnittstelle ist bei dem persönlichen medizinischen Gerät vorgesehen.

Insbesondere für eine derartige Konstellation ist es vorteilhaft, wenn das programmierbare persönliche Gerät dazu ausgebildet ist, einen empfangenen Programmierauftrag an das ihm zugeordnete Implantat weiterzuleiten und mit jedem Weiterleiten eine Weiterleitungsbestätigung als Programmierbestätigung an das Servicecenter abzusenden.

Ebenso ist es vorteilhaft, wenn das persönliche medizinische Gerät bereits bei Empfang eines Programmierauftrags seitens des Servicecenters eine Empfangsbestätigung als Programmierbestätigung an das Servicecenter absendet. In beiden Fällen ist das persönliche medizinische Gerät vorzugsweise dazu ausgebildet, der jeweiligen Programmierbestätigung einen Zeitstempel in Form von dem Zeitpunkt einer Weiterleitung des Programmierauftrags oder den Zeitpunkt des Empfangs des Programmierauftrags beschreibende Daten hinzuzufügen.

Entsprechend ist es vorteilhaft, wenn das Implantat ebenfalls dazu ausgebildet ist, nach Empfang eines Programmierauftrags seitens des persönlichen medizinischen Gerätes eine Empfangsbestätigung über das persönliche medizinische Gerät an das Servicecenter zu senden. Das persönliche medizinische Gerät ist dabei dazu ausgebildet, diese Empfangsbestätigung des Implantats an das Servicecenter weiterzuleiten.

Das Implantat wiederum ist dazu ausgebildet, nach Empfang eines Programmierauftrags diesen nach Möglichkeit auszuführen, d. h. eine entsprechende Programmierung seiner selbst zu veranlassen. Ist diese erfolgreich, sendet das Implantat vorzugsweise eine Erfolgsmeldung über das programmierbare persönliche Gerät an das Servicecenter. Sollte das Ausführen des Programmierauftrags, also die Installation des entsprechenden Programms nicht erfolgreich sein, sendet das Implantat eine Misserfolgsmeldung über das persönliche medizinische Gerät an das Servicecenter.

Das Servicecenter ist dazu ausgebildet, Programmierbestätigungen der verschiedenen Art entgegenzunehmen. Dazu weist das Servicecenter vorzugsweise eine Datenbank auf, die Einträge über den jeweiligen Status des Programmierauftrags samt der zugehörigen Zeitstempel enthält. Vorzugsweise sind in der Datenbank nicht jeweils nur der letzte Status, sondern mehrere vorangegangene Status mit dem zugehörigen Zeitstempel gespeichert.

Dabei kann das Servicecenter bzw. dessen programmierbare Überwachungseinheit gemäß einer bevorzugten Ausführungsvariante dazu ausgebildet sein, für die verschiedenen Übertragungsschritte, die ein Programmierauftrag zu durchlaufen hat, jeweils eigene Programmierschlusszeitpunkte festzusetzen und zu überwachen.

Sobald einer dieser Programmierschlusszeitpunkte überschritten wird, ohne dass zuvor die dazugehörige Programmierbestätigung bei dem Servicecenter eingetroffen ist, sendet das Servicecenter, ausgelöst durch die programmierbare Überwachungseinheit einen entsprechenden Programmierlöschauftrag aus, der bewirkt, dass der zuvor abgesandte Programmierauftrag gelöscht wird.

Das Servicecenter und die dazu gehörige Programmierüberwachungseinheit ist vorzugsweise dazu ausgebildet, mehrere Programmieraufträge gleichzeitig zu überwachen. In diesem Zusammenhang ist es vorteilhaft, wenn es die Benutzerschnittstelle erlaubt, dass ein Benutzer eines oder mehrere von Implantaten auswählt, die über das Servicecenter und entsprechende programmierbare persönliche Geräte zugänglich sind.

Hinsichtlich der Festlegung eines Programmierabschlusszeitpunktes bestehen grundsätzlich mehrere Varianten. Ein jeweiliger Programmierabschlusszeitpunkt kann:
- individuell vom Arzt für jeden Umprogrammier-Auftrag festlegbar sein,
- individuell vom Arzt für jeden Patienten festlegbar sein,
- individuell vom Arzt für alle seine Patienten festlegbar sein,
- individuell vom Arzt für jedes Implantat-Modell festlegbar sein,
- individuell vom Arzt für jede Produktfamilie festlegbar sein,
- individuell vom Arzt für jede Produkttyp (Tachy-, Brady- oder CRT-Gerät o.ä.) festlegbar sein,
- individuell vom Arzt für jedes Krankheitsbild, das dem Implantatträger zugeordnet ist, festlegbar sein,
- individuell vom Arzt für jede Therapieart, die dem Implantatträger zugeordnet ist, festlegbar sein,
- individuell vom Arzt je nach Inhalt des Umprogrammierauftrags festlegbar sein,
oder aber fest im Servicecenter oder durch die Fernprogrammieranwendung vorgegeben sein oder gemäß der oben genannten Kategorien im Servicecenter oder durch die Femprogrammieranwendung vorgegeben sein.

Eine Lösung der Aufgabe besteht auch in einem Servicecenter für die zuvor beschriebene Anordnung. Das Service Center besitzt eine Datenkommunikationsschnittstelle zum wenigstens mittelbaren Verbinden des Service Centers mit dem programmierbaren persönlichen Gerät und eine Benutzschnittstelle, die ausgebildet ist, Programmieraufträge für das programmierbare persönliche Gerät zusammenzustellen und ein Absenden eines zusammengestellten Programmierauftrags auszulösen. Außerdem besitzt das Service Center eine Programmierüberwachungseinheit, die ausgebildet ist, einen vom Zeitpunkt des Absendens des Programmierauftrags abhängigen Programmierschlusszeitpunkt zu bestimmen und den Programmierauftrag als fehlgeschlagen anzusehen, falls das Service Center bis zum Programmierschlusszeitpunkt keine eine erfolgreiche Ausführung des Programmierauftrags bestätigende Programmierbestätigung seitens des programmierbaren persönlichen Gerätes empfangen hat.

Vorzugsweise ist die Programmierüberwachungseinheit mit einer Datenbank verbunden und ausgebildet ist, in der Datenbank Einträge für mehrere Programmierschlusszeitpunkte für ein oder mehrere persönliche Geräte und/der ein oder mehrere Übertragungs- oder Ausführungsschritte eines Programmierauftrags vorzunehmen sowie entsprechende Programmierbestätigungen mit Zeitstempel den Datenbankeinträgen zuzuordnen. Besonders vorteilhaft ist es, wenn die Benutzerschnittstelle als Fernprogrammieranwendung ausgebildet ist, die in Abhängigkeit einer Fernprogrammierservereinheit auf einem von dem Service Center entfernten Gerät lauffähig ist und die ausgebildet ist, eine Programmierung mehrerer unterschiedlicher persönlicher Geräte zu erlauben und eine Eingabeschnittstelle zur Auswahl eines Gerätes und zum Zusammenstellen eines Programmierauftrags für ein jeweils ausgewähltes persönliches Gerät aufweist. Dabei kann die Femprogrammieranwendung eine Eingabeeinheit für das Eingeben eines oder mehrerer Programmierschlusszeitpunkte zu einem jeweiligen Programmierauftrag aufweisen.

Eine weitere Lösung der Aufgabe bestehet in einem Verfahren zum Fernprogrammieren eines programmierbaren persönlichen medizinischen Gerätes, insbesondere eines implantierbaren medizinischen Gerätes wie ein Herzschrittmacher, Defibrillator oder dergleichen, mittels einer Anordnung der vorbeschriebenen Art. Das Verfahren umfasst die Verfahrensschritte:
- Auswählen eines zu programmierenden persönlichen Gerätes
- Zusammenstellen eines Programmierauftrags für das ausgewählte persönliche Gerät,
- Optional das Bestimmen mindestens eines Programmierschlusszeitpunktes für den Programmierauftrag, bis zu dem ein Übertragungsschritt des Programmierauftrags und/oder ein Ausführen des Programmierauftrags abgeschlossen sein sollen
- Optional das Hinterlegen eines fixen Programmierschlusszeitpunkts im System bis zu dem ein Übertragungsschritt des Programmierauftrags und/oder ein Ausführen des Programmierauftrags abgeschlossen sein sollen und der standardmäßig für alle Umprogrammierungen verwendet wird.
- Überwachen eines jeweiligen Programmierschlusszeitpunktes durch Empfangen und Auswerten von entsprechenden Programmierbestätigungen, und
- automatische Misserfolgsmarkierung eines Programmierauftrags, falls eine zu einem zugehörigen Programmierschlusszeitpunkt gehörende Programmierbestätigung nicht bis zu diesem Programmierschlusszeitpunkt empfangen wird.

Eine alternative Lösung der Aufgabe besteht darin, dass das Servicecenter beim Absenden des Programmierauftrags diesem Daten mit einem oder mehreren Programmierschlusszeitpunkten hinzufügt. In dieser Konstellation sind die an der Übertragung des Programmierauftrags beteiligten Geräte dazu ausgebildet, anhand der dem Programmierauftrag beigefügten Programmierschlusszeitpunkt zu überprüfen, ob der Programmierauftrag rechtzeitig beim jeweiligen Gerät eingetroffen ist, also vor dem für das jeweilige Gerät gültigen Programmierschlusszeitpunkt. Falls dies nicht der Fall ist, ist das jeweilige Gerät dazu ausgebildet, den Programmierauftrag nicht weiterzuleiten oder auszuführen, sondern zu löschen. Der letzte der Programmierschlusszeitpunkte definiert dabei den für ein jeweiliges Implantat gültigen Programmierschlusszeitpunkt, bis zu dem die erfolgreiche Ausführung des jeweiligen Programmierauftrags erfolgt sein muss.

Weitere vorteilhafte Ausgestaltungen der Erfindung ergeben sich durch Kombination von Merkmalen der Ansprüche sowie aus der nachfolgenden Beschreibung eines bevorzugten Ausführungsbeispiels.

Die Erfindung soll nun anhand eines bevorzugten Ausführungsbeispiels mit Bezug auf die Figuren näher erläutert werden. Diese zeigen in:
- Fig. 1:: eine Übersicht über eine Anordnung zur Fernprogrammierung von Implantaten;
- Fig. 2:: Blockschaltbilder der wesentlichen Komponenten Implantat, Patientengerät und Servicecenter;
- Fig. 3:: eine schematische Darstellung einer Fernprogrammierung eines Implantats im Erfolgsfall; und
- Fig.4 :: eine schematische Darstellung einer Fernprogrammierung eines Implantats im Fehlerfall.

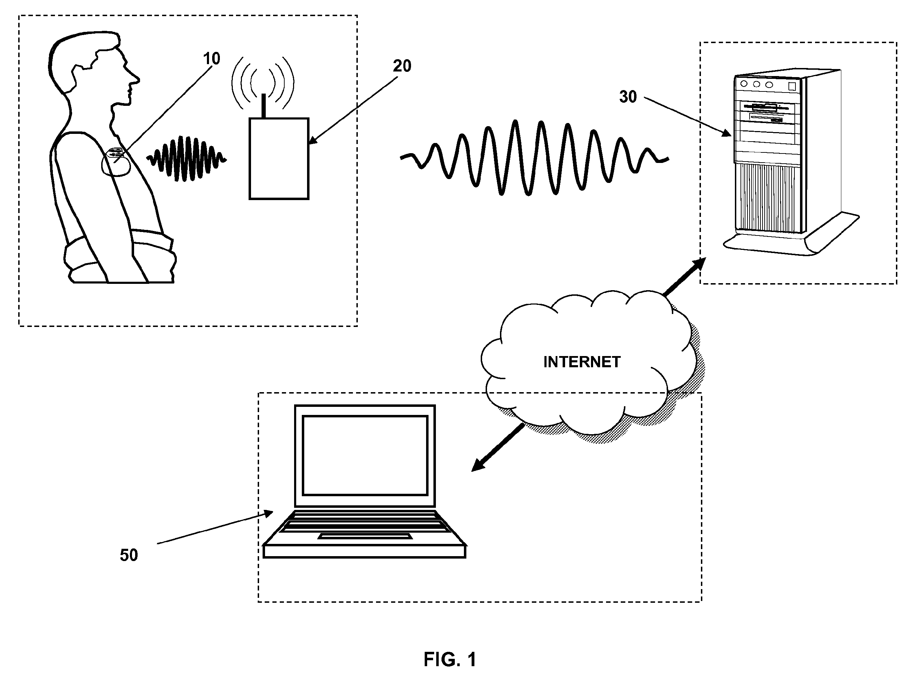

Die in Fig. 1 abgebildete Anordnung zur Fernprogrammierung eines Implantats 10 umfasst als weitere Komponenten:

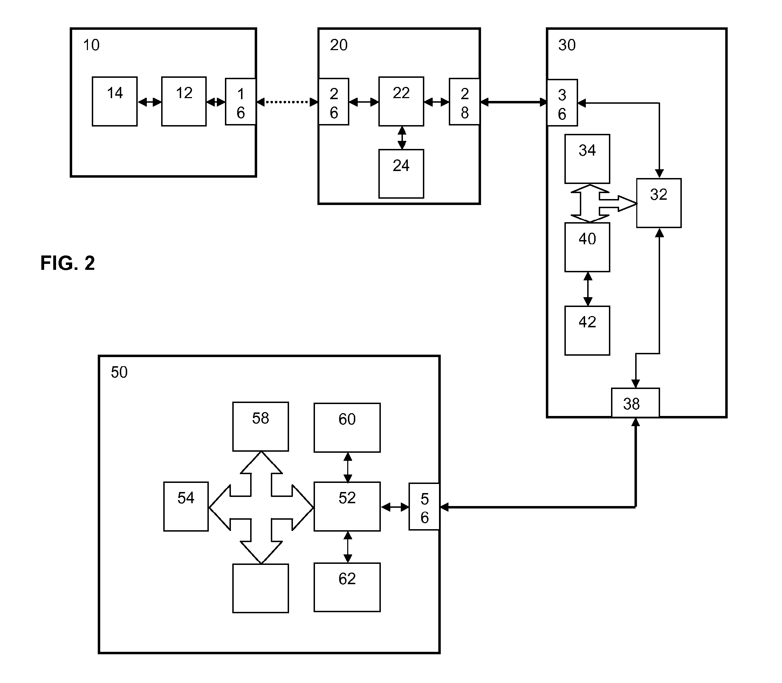

Figur 1 zeigt die Komponenten einer beispielhaften Anordnung zur Fernprogrammierung von Implantaten. Eine Komponente ist das Implantat 10 selbst. Dieses besitzt eine Steuerung 12 (siehe Figur 2), die sowohl mit einem Speicher 14 als auch mit einer Telemetrieeinheit 16 verbunden ist. Die Telemetrieeinheit 16 ermöglicht eine drahtlose, bidirektionale Datenübertragung von und zu dem Implantat 10.

Eine weitere Komponente ist ein Patientengerät 20, das ebenfalls eine Steuerung 22 aufweist, die mit einem Speicher 24 und einer ersten Telemetrieeinheit 26 für eine bidirektionale, Datenübertragung von und zu dem Implantat 10 verbunden ist sowie mit einer zweiten Datenkommunikationsschnittstelle 28, über die das Patientengerät 20 bidirektional Daten mit einem zentralen Servicecenter 30 austauschen kann.

Das zentrale Servicecenter 30 weist neben einer Steuerung 32 eine zentrale Datenbank 34 und eine erste Datenkommunikationsschnittstelle 36 auf, über die das Servicecenter 30 bidirektional Daten mit dem Patientengerät 20 austauschen kann. Außerdem weist das zentrale Servicecenter 30 eine Fernprogrammierservereinheit 40 auf, die sowohl mit der Steuereinheit 32 des Servicecenters verbunden ist als auch mit einer Internetschnittstelle 38 zum Anschluss eines Fernprogrammierclients 50.

Der Fernprogrammierclient 50 besitzt ebenfalls eine zentrale Steuereinheit 52, die mit einem Speicher 54 und einer Datenkommunikationsschnittstelle 56 für eine Datenübertragung von und zum Service Center 30 verbunden ist. Außerdem weist der Fernprogrammierclient 50 eine Fernprogrammieranwendung 58 auf, die mit Hilfe der zentralen Steuereinheit 52 und des Speichers 54 ausgeführt werden kann und entweder als stand-alone Lösung ausschließlich auf den Fernprogrammierclient 50 lauffähig ist oder als Serveranwendung teilweise oder ganz auch auf das zentrale Servicecenter 30 und deren Fernprogrammierservereinheit 40 zurückgreift.

Die Fernprogrammieranwendung 58 erlaubt es, auf den Fernprogrammierclient 50 Programme zusammenzustellen, die für das Implantat 10 und dessen Steuerung 12 ausführbar sind. Die so zusammengestellten Programme können in Form eines Programmierauftrages von dem zentralen Servicecenter 30 über das Patientengerät 20 zum Implantat 10 übertragen werden.

Um von der Fernprogrammieranwendung generierte Darstellungen anzuzeigen, weist der Fernprogrammierclient 50 eine Anzeige 60 auf. Damit ein Benutzer, beispielsweise ein Arzt, Eingaben für die Fernprogrammierung des Implantats 10 machen kann, ist außerdem eine Eingabeeinheit 62 vorgesehen.

Räumlich befinden sich das Implantat 10 (ohnehin) und das Patientengeräts 20 in der Nähe eines Patienten. Das zentrale Servicecenter 30 ist an einem zentralen Ort angeordnet. Der Fernprogrammierclient 50 befindet sich in der Nähe eines Arztes und kann vom Servicecenter 30 weit entfernt sein.

Wie eingangs beschrieben, besteht bei einer Fernprogrammierung des Implantates 10 mit Hilfe der Fernprogrammieranwendung 56 über das Servicecenter 30 und das Patientengerät 20 das Problem, dass die Übertragung eines mit der Fernprogrammieranwendung 56 erstellten Programmierauftrags gegebenenfalls auch eine längere Zeit in Anspruch nehmen kann. Beispielsweise kann es passieren, dass sich das Implantat für einen gewissen Zeitraum nicht in der Nähe des Patientengerätes befindet oder dass das Servicecenter aus irgendwelchen Gründen keine Verbindung zum Patientengerät herstellen kann. Dabei kann es auch vorkommen, dass ein für eine bestimmte Situation maßgeschneiderter Programmierauftrag nicht mehr angemessen ist, wenn er nach verzögerter Übertragung zum Implantat nicht mehr aktuell ist. Um dieses Problem zu vermeiden, besitzt die Fernprogrammier-Servereinheit 40 eine Programmierüberwachungseinheit 42, die jedem Programmierauftrag einen Programmierschlusszeitpunkt zuordnet.

Das Patientengerät 20 und das Implantat 10 sind so ausgebildet, dass sie jeweils den Empfang eines Programmierauftrags mit einer Programmierbestätigung im Sinne einer Empfangsbestätigung quittieren. Diese Programmierbestätigung wird jeweils von dem Patientengerät 20 oder dem Implantat 10 an das Servicecenter 30 zurückgesandt. Dabei fügt jedes Gerät, also Patientengerät 20 oder Implantat 10 der jeweiligen Programmierbestätigung einen Zeitstempel in Form von Daten hinzu, die den Empfangszeitpunkt des Programmierauftrags wiedergeben. Die Fernprogrammier-Servereinheit 40 speichert den zu einem jeweiligen Programmierauftrag empfangenen Programmierbestätigungen samt der zugehörigen Kennung eines jeweiligen Gerätes und des zugehörigen Zeitstempels in der Datenbank 34.

Wenn das Implantat schließlich einen empfangenen Programmierauftrag ausgeführt hat und beispielsweise eine Programmierung oder Umprogrammierung seiner selbst durchgeführt hat, sendet das Implantat 10 eine Programmierbestätigung im Sinne einer Erfolgsmeldung (oder ggf. auch einer Misserfolgsmeldung) zurück an das Servicecenter 30. Dort wird diese Programmierbestätigung ebenfalls von der Fernprogrammier-Servereinheit 40 verarbeitet und der Zeitstempel, der die Erfolgsmeldung enthaltende Programmierbestätigung mit dem Programmierschlusszeitpunkt für den jeweiligen Programmierauftrag verglichen.

Parallel dazu überwacht die Programmierüberwachungseinheit 42 der Fernprogrammier-Servereinheit 40 regelmäßig die Programmierschlusszeitpunkte für einzelne Programmieraufträge. Sobald einer dieser Programmierschlusszeitpunkte erreicht ist und die zugehörige Erfolgsmeldung des entsprechenden Implantates noch nicht vorliegt, löst die Fernprogrammier-Überwachungseinheit einen Löschungsauftrag aus, der so gestaltet ist, dass ein Löschen des noch nicht ausgeführten Programmierauftrags bewirkt. Damit wird sichergestellt, dass ein Programmierauftrag nicht zur Unzeit ausgeführt wird.

Alternativ dazu kann die Fernprogrammier-Servereinheit 40 auch so ausgebildet sein, dass sie einem jeweiligen Programmierauftrag Daten hinzufügt, die den Programmierschlusszeitpunkt beschreiben. Sowohl das Patientengerät 20 als auch das Implantat 10 können dazu ausgebildet sein, durch eigene Steuereinheiten zu prüfen, ob der Programmierschlusszeitpunkt eines jeweils empfangenen Programmierauftrags bereits erreicht ist. Falls dies der Fall ist, löscht das Patientengerät 20 über das Implantat den Programmierauftrag und leitet ihn nicht weiter bzw. führt in nicht aus.

Bezüglich der Festlegung des Programmierschlusszeitpunktes bestehen verschiedene Varianten. Über die Fernprogrammieranwendung 58 kann ein jeweiliger Programmierschlusszeitpunkt vom Arzt selbst festgelegt sein. In diesem Falle ist die Femprogrammieranwendung 58 so ausgelegt, dass sie eine entsprechende Eingabe erlaubt. Die Femprogrammieranwendung 58 kann weiterhin so ausgebildet sein, mit ihrer Hilfe feste Programmierschlusszeitpunkt für ein jeweiliges Implantat festzulegen sind. Dies ist besonders dann sinnvoll, wenn ein Arzt nicht nur ein, sondern mehrere Implantate betreut. Die Femprogrammieranwendung 58 kann auch so ausgebildet sein, dass ein Arzt einen Programmierschlusszeitpunkt - jeweils vom Absenden des Programmierauftrags an gerechnet - für alle von ihm betreuten Implantate festlegt.

Eine weitere Alternative besteht darin, dass die Fernprogrammieranwendung 58 so gestaltet ist, dass sie es erlaubt, verschiedene Programmierschlusszeitpunkte für unterschiedliche Implantatmodelle oder Modellfamilien vorzugeben.

Eine weitere Alternative besteht darin, dass die Fernprogrammieranwendung 58 er erlaubt, unterschiedliche Programmierschlusszeitpunkte für verschiedene Produkttypen, beispielsweise Herzschrittmacher oder Defibrillator, vorzugeben oder für unterschiedliche Arten von Programmen.

Darüber hinaus kann die Fernprogrammier-Servereinheit 40 ausgebildet sein, für einen jeweiligen Programmierauftrag einen geeigneten Programmierschlusszeitpunkt automatisch zu bestimmen. Hierzu können in der Datenbank 34 entsprechende Daten, beispielsweise individuellen Patienten oder Geräten zugeordnet, hinterlegt sein.

Insbesondere ist es möglich, dass die Fernprogrammier-Servereinheit 40 einem Programmierauftrag mehrere Programmierschlusszeitpunkte zuweist, die den unterschiedlichen Geräten bzw. Übertragungs- und Verarbeitungsschritten eines jeweiligen Programmierauftrags zugeordnet sind. Beispielsweise kann das es zuvor beschriebenen Beispiel möglich sein, einen Programmierschlusszeitpunkt für den Empfang der Programmierbestätigung mit der Empfangsbestätigung des Patientengerätes 20 festzulegen, einen anderen, späteren Programmierschlusszeitpunkt für den Empfang der Programmierbestätigung und Empfangsbestätigung des Implantates 10 und schließlich einen dritten, noch späteren Programmierschlusszeitpunkt für den Empfang der Programmierbestätigung mit der Erfolgsmeldung des Implantates 10.

Im Folgenden sind einige Varianten für eine Bestimmung des Programmierschlusszeitpunktes aufgelistet. Der Programmierschlusszeitpunkt kann:
- individuell vom Arzt für jeden Umprogrammier-Auftrag festlegbar sein,
- individuell vom Arzt für jeden Patienten festlegbar sein,
- individuell vom Arzt für alle seine Patienten festlegbar sein,
- individuell vom Arzt für jedes Implantat-Modell festlegbar sein,
- individuell vom Arzt für jede Produktfamilie festlegbar sein,
- individuell vom Arzt für jede Produkttyp (Tachy-, Brady- oder CRT-Gerät o.ä.) festlegbar sein,
- individuell vom Arzt für jedes Krankheitsbild, das dem Implantatträger zugeordnet ist, festlegbar sein,
- individuell vom Arzt für jede Therapieart, die dem Implantatträger zugeordnet ist, festlegbar sein,
- individuell vom Arzt je nach Inhalt des Umprogrammierauftrags festlegbar sein,
oder aber fest im Servicecenter oder durch die Fernprogrammieranwendung vorgegeben sein oder gemäß der oben genannten Kategorien im Servicecenter oder durch die Femprogrammieranwendung vorgegeben sein.

Alternativ kann es für jede unten beschriebene Teilstrecke der Datenübermittlung separate Programmierschlusszeitpunkte geben. Beispielsweise kann der Programmierschlusszeitpunkt für die Übertragung vom Servicecenter bis zum Patientengerät 24 h zulassen und der weitere Programmierschlusszeitpunkt für die Übertragung vom Patientengerät zum Implantat 72 h.

Die Programmierung eines Implantats 10 erfolgt mit Hilfe der hier beschriebenen Anordnung beispielsweise wie folgt:
1. Der Arzt wählt mit Hilfe der (Web-)Anwendung Servicecenter ein Implantat aus stellt ein neues/modifiziertes Programm für dieses zusammen.
2. Der Arzt sendet dieses Programm als Auftrag für eine Umprogrammierung aus der Ferne ab.
3. Das Servicecenter fügt dem erhaltenen Auftrag den voreingestellten Programmierschlusszeitpunkt hinzu.
4. Das Servicecenter sendet diesen Auftrag über eine bestehende Datenverbindung (z. B. GPRS, UMTS, GSM, Modem o.ä.) an das Patientengerät und speichert sich den Zeitstempel dieser Sendung sowie den aktuellen Status (in etwa "Auftrag an Patientengerät übermittelt").
5. Das Patientengerät sendet eine Programmierbestätigung über den Erhalt des Auftrags zurück an das Servicecenter, welches sich wiederum den Zeitstempel des Erhalts der Programmierbestätigung sowie den aktuellen Status speichert (in etwa "Erhalt des Auftrags vom Patientengerät bestätigt.").
6. Das Patientengerät versucht nun, den Auftrag über eine Funkdatenverbindung an das Implantat zu übermitteln und informiert das Servicecenter über jeden dieser Versuche mit einer entsprechenden Programmierbestätigung. Die Übermittlung an das Implantat kann nach einem gewissen Schema wiederholt werden.
7. Das Servicecenter speichert den Zeitstempel der Versuche des Patientengerätes, den Auftrag an das Implantat zu übertragen sowie den aktuellen Status (in etwa "Übermittlung des Umprogrammier-Auftrags an das Implantat erfolgreich/nicht erfolgreich") in der Datenbank.
8. Das Implantat empfängt den Umprogrammier-Auftrag und bestätigt den Empfang an das Patientengerät.
9. Das Implantat versucht, das erhaltene Programm zu aktivieren und übersendet eine Programmierbestätigung über den Erfolg oder Misserfolg dieser Aktion via Patientengerät an das Servicecenter.
10. Das Servicecenter speichert den Zeitstempel und den Status der Aktivierung des mit dem Programmierauftrag übertragenen Programms (in etwa "Aktivierung des Umprogrammier-Auftrags erfolgreich/nicht erfolgreich.") in der Datenbank.
11. Der Arzt hat zu jedem Zeitpunkt die Möglichkeit, sich mit Hilfe der Femprogrammieranwendung über eine Abfrage der Datenbank 34 über den aktuellen Status des Umprogrammier-Auftrags zu informieren.

Schritte 4-9 werden nur ausgeführt, sofern der im Umprogrammier-Auftrag enthaltene Programmierschlusszeitpunkt noch nicht abgelaufen ist. Sobald der Programmierschlusszeitpunkt abgelaufen ist, verwirft diejenige Komponente, die gerade im Besitz des Auftrags ist, diesen und informiert das Servicecenter darüber. Daraufhin speichert das Servicecenter den Zeitstempel und den Status (in etwa "Zeitspanne für die Gültigkeit des Umprogrammier-Auftrags abgelaufen").

Die Fortschreibung der Status der Übermittlung des Umprogrammier-Auftrags kann auf eine Teilmenge an wesentlichen Status begrenzt sein. So kann beispielsweise auf die Übertragung des Status "Patientengerät hat Auftrag zum siebten Mal nicht erfolgreich an das Implantat übertragen können" verzichtet werden.

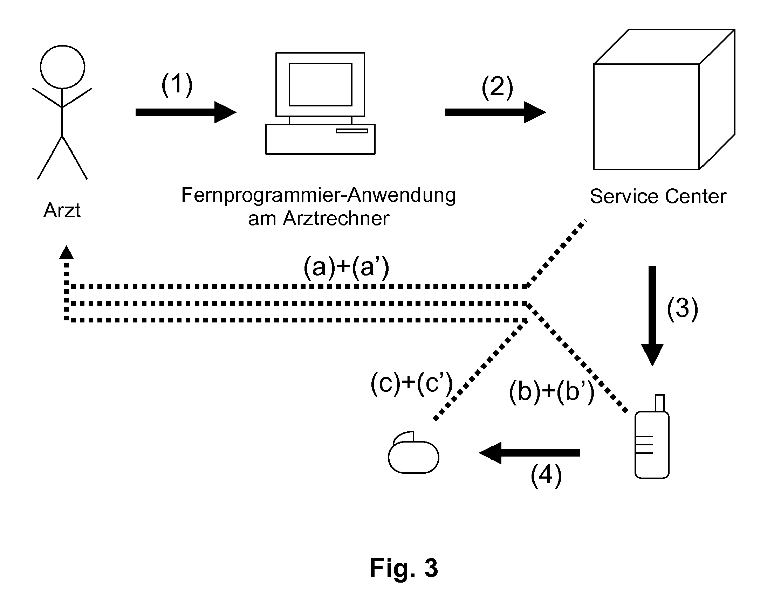

Dies ist für den Erfolgsfall in Figur 3 noch einmal dargestellt.
- Schritt (1):: Der Arzt wählt ein Implantat aus und stellt ein Programm dafür zusammen.
- Schritt (2): Das Programm wird vom Rechner des Arztes an das Servicecenter übertragen.
- Schritt (a): Das Servicecenter bestätigt der Fernprogrammier-Anwendung den korrekten Erhalt.
- Schritt (3): Der Servicecenter leitet die Umprogrammierung an das Patientengerät. weiter.
- Schritt (a'): Der Servicecenter bestätigt dem Arzt die korrekte erfolgte Weiterleitung an das Patientengerät.
- Schritt (4): Das Patientengerät leitet die Umprogrammierung an das Implantat weiter.
- Schritt (c): Das Implantat bestätigt dem Arzt den korrekten Erhalt der Umprogrammierung und später
- Schritt (c'): auch die korrekte erfolgte Umprogrammierung.

Alle Bestätigungen und Statusrückinformationen erfolgen in derselben Kette, in der die Information selbst gelaufen ist, nur rückwärts.

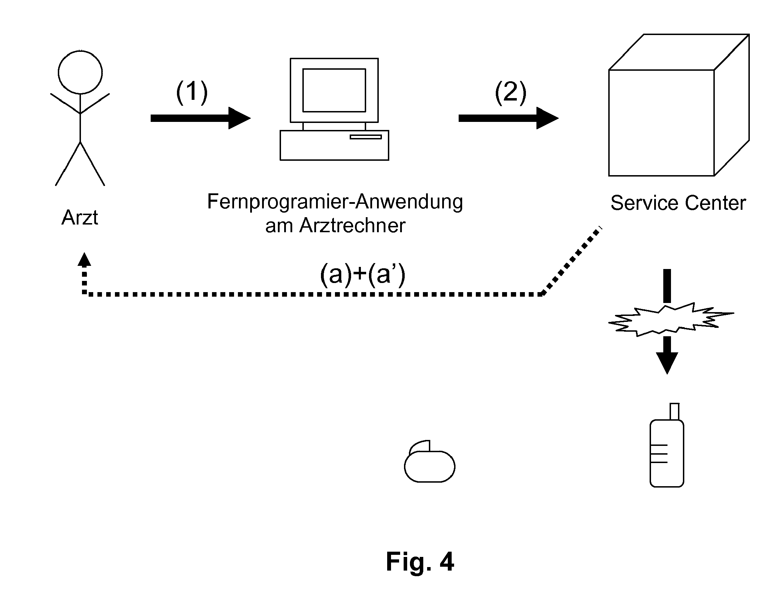

Figur 4 zeigt den Ablauf im Falle einer gescheiterten Übertragung:
- Schritt (1): Der Arzt wählt ein Implantat aus und stellt ein Programm dafür zusammen.
- Schritt (2): Das Programm wird vom Rechner des Arztes an das Servicecenter übertragen.
- Schritt (a): Das Servicecenter bestätigt der Fernprogrammier-Anwendung den korrekten Erhalt.
- Schritt (3): Das Servicecenter leitet die Umprogrammierung an das Patientengerät. weiter. Dies schlägt fehl (z.B., weil das Patientengerät nicht erreichbar ist oder weil es den Erhalt nicht vor Eintreten eines vorgesehenen Programmierschlusszeitpunkts bestätigt.
- Schritt (a'): Das Servicecenter teilt dem Arzt mit, dass die Nachricht nicht weitergeleitet werden konnte und die Umprogrammierung gescheitert ist.

Die Fernprogrammieranwendung ist derart ausgebildet, dass ein Arzt mehrere Implantate betreuen kann. Entsprechend besitzt sie eine Eingabeeinheit, mit der ein jeweiliges, zu programmierendes Implantat ausgewählt und ein Programmierauftrag für dieses Implantat zusammengestellt werden kann.

## Patentansprüche

1. Anordnung für die Fernprogrammierung eines programmierbaren persönlichen medizinischen Gerätes mit den Komponenten:
- programmierbares persönliches Gerät (10; 20) und
- Servicecenter (30)
von denen das programmierbare persönliche Gerät
eine Datenkommunikationsschnittstelle zum wenigstens mittelbaren Verbinden des persönlichen programmierbaren Gerätes mit dem Servicecenter und
eine programmierbare Steuerung für Funktionen des persönlichen Gerätes aufweist, und
von denen das Servicecenter (30)
eine Datenkommunikationsschnittstelle zum wenigstens mittelbaren Verbinden des Servicecenters mit dem programmierbaren persönlichen Gerät und
eine Benutzschnittstelle aufweist, die ausgebildet ist, Programmieraufträge für das programmierbare persönliche Gerät zusammenzustellen und ein Absenden eines zusammengestellten Programmierauftrags auszulösen,
**dadurch gekennzeichnet, dass**
das Servicecenter (30) eine Programmierüberwachungseinheit (40) aufweist, die ausgebildet ist, einen vom Zeitpunkt des Absendens des Programmierauftrags abhängigen Programmierschlusszeitpunkt zu bestimmen und den Programmierauftrag zu stornieren oder zu löschen, falls das Servicecenter bis zum Programmierschlusszeitpunkt keine eine erfolgreiche Ausführung des Programmierauftrags bestätigende Programmierbestätigung seitens des programmierbaren persönlichen Gerätes empfangen hat, und
das programmierbare persönliche Gerät dazu ausgebildet ist, nach Empfang eines Programmierauftrags oder nach erfolgreicher Weiterleitung eines Programmierauftrags eine Programmierbestätigung an das Servicecenter abzusenden.

2. Anordnung nach Anspruch 1, **dadurch gekennzeichnet, dass** das persönliche programmierbare Gerät ein Patientengerät (20) ist, welches neben der Datenkommunikationsschnittstelle zum wenigstens mittelbaren Verbinden des persönlichen programmierbaren Gerätes mit dem Servicecenter eine kurzreichweitige Schnittstelle für eine Datenkommunikation mit einem Implantat aufweist.

3. Anordnung nach Anspruch 2, **dadurch gekennzeichnet, dass** das programmierbare persönliche Gerät dazu ausgebildet ist,
- einen empfangenen Programmierauftrag an ein dem programmierbaren persönlichen Gerät zugeordnetes Implantat weiterzuleiten und mit jedem Weiterleiten eine Weiterleitungsbestätigung als Programmierbestätigung an das Servicecenter abzusenden, und / oder
- nach Empfang eines Programmierauftrags eine Empfangsbestätigung als Programmierbestätigung an das Servicecenter abzusenden und / oder
- einen Programmierauftrag zu empfangen und daraufhin eine Empfangsbestätigung als Programmierbestätigung abzusenden.

4. Anordnung nach Anspruch 3, **dadurch gekennzeichnet, dass** das programmierbare persönliche Gerät dazu ausgebildet ist, der Programmierbestätigung einen Zeitstempel in Form des Zeitpunkts der Weiterleitung des Programmierauftrags oder den Zeitpunkt des Empfangs des Programmierauftrags beschreibenden Daten hinzuzufügen.

5. Anordnung nach einem der Ansprüche 1 bis 4, **dadurch gekennzeichnet, dass** Implantat (10) eine weitere Komponente der Anordnung neben dem Patientengerät (20) ist, das eine Datenkommunikationsschnittstelle aufweist, die für eine bidirektionale Datenkommunikation mit dem Patientengerät ausgebildet ist, wobei
- das Implantat (10) ausgebildet ist, einen Programmierauftrag seitens des Patientengerätes (20) zu empfangen und daraufhin eine Empfangsbestätigung an das Patientengerät abzusenden und
das Patientengerät (20) ausgebildet ist, eine Empfangsbestätigung des Implantats (10) als Programmierbestätigung an das zentrale Servicecenter weiterzuleiten, und / oder
- eine Programmierauftrag auszuführen und im Falle eines erfolgreichen Ausführens eine Erfolgsmeldung an das programmierbare persönliche Gerät zu senden und das programmierbare persönliche Gerät ausgebildet ist, eine Erfolgsmeldung des Implantats als Programmierbestätigung an das zentrale Servicecenter weiterzuleiten, und / oder
- im Falle eines nicht erfolgreichen Ausführens eine Misserfolgsmeldung an das programmierbare persönliche Gerät zu senden und das programmierbare persönliche Gerät ausgebildet ist, eine Misserfolgsmeldung des Implantats als Programmierbestätigung an das zentrale Servicecenter weiterzuleiten.

6. Anordnung nach Anspruch 5, **dadurch gekennzeichnet, dass** entweder das Implantat oder das programmierbare persönliche Gerät ausgebildet ist, einer Empfangsbestätigung des Implantats, einer Erfolgsmeldung oder einer Misserfolgsmeldung einen Zeitstempel hinzuzufügen.

7. Anordnung nach einem der Ansprüche 1 bis 6, **dadurch gekennzeichnet, dass** die Programmierüberwachungseinheit dazu ausgebildet ist,
- den Status der Übermittlung und Ausführung eines Programmierauftrages zu speichern und anhand eintreffender Programmierbestätigen zu aktualisieren und/oder
- nicht nur den letzten Status der Übertragung und Ausführung des Programmierauftrags zu speichern, sondern auch vorangegangene Status zusammen mit dem jeweilig dazugehörenden Zeitstempel.

8. Servicecenter für eine Anordnung gemäß einem der Ansprüche 1 bis 7, **dadurch gekennzeichnet, dass** das Servicecenter (10)
eine Datenkommunikationsschnittstelle (36) zum wenigstens mittelbaren Verbinden des Servicecenters mit dem programmierbaren persönlichen Gerät und
eine Benutzschnittstelle aufweist, die ausgebildet ist, Programmieraufträge für das programmierbare persönliche Gerät zusammenzustellen und ein Absenden eines zusammengestellten Programmierauftrags auszulösen,
**dadurch gekennzeichnet, dass**
das Servicecenter eine Programmierüberwachungseinheit (40) aufweist, die ausgebildet ist, einen vom Zeitpunkt des Absendens des Programmierauftrags abhängigen Programmierschlusszeitpunkt zu bestimmen und den Programmierauftrag zu löschen, falls das Servicecenter bis zum Programmierschlusszeitpunkt keine eine erfolgreiche Ausführung des Programmierauftrags bestätigende Programmierbestätigung seitens des programmierbaren persönlichen Gerätes empfangen hat.

9. Servicecenter nach Anspruch 8, **dadurch gekennzeichnet, dass** die Programmierüberwachungseinheit (40) mit einer Datenbank (34) verbunden und ausgebildet ist, in der Datenbank Einträge für mehrere Programmierschlusszeitpunkte für ein oder mehrere persönliche Geräte und/der ein oder mehrere Übertragungs- oder Ausführungsschritte eines Programmierauftrags vorzunehmen sowie entsprechende Programmierbestätigungen mit Zeitstempel den Datenbankeinträgen zuzuordnen.

10. Servicecenter nach einem der Ansprüche 8 oder 9, **dadurch gekennzeichnet, dass** die Benutzerschnittstelle als Fernprogrammieranwendung (58) ausgebildet ist, die in Abhängigkeit einer Fernprogrammierservereinheit auf einem vom Servicecenter (40) entfernten Gerät (50) lauffähig ist und die ausgebildet ist, eine Programmierung mehrerer unterschiedlicher persönlicher Geräte zu erlauben und eine Eingabeschnittstelle zur Auswahl eines Gerätes und zum Zusammenstellen eines Programmierauftrags für ein jeweils ausgewähltes persönliches Gerät aufweist.

11. Servicecenter nach Anspruch 10, **dadurch gekennzeichnet, dass** die Femprogrammieranwendung eine Eingabeeinheit für das Eingeben eines oder mehrerer Programmierschlusszeitpunkte zu einem jeweiligen Programmierauftrag aufweist.

12. Programmierbares, persönliches Gerät, insbesondere Herzschrittmacher und/oder Kardioverter/Defibrillator, für eine Anordnung gemäß einem der Ansprüche 1 bis 7, **dadurch gekennzeichnet, dass** das persönliche Gerät
eine Datenkommunikationsschnittstelle zum wenigstens mittelbaren Verbinden des persönlichen programmierbaren Gerätes mit dem Servicecenter und eine programmierbare Steuerung für Funktionen des persönlichen Gerätes aufweist, und ausgebildet ist
nach Empfang, Weiterleiten oder Ausführen eines Programmierauftrag eine entsprechende Programmierbestätigung an das Servicecenter abzusenden.

13. Verfahren zum Fernprogrammieren eines programmierbaren persönlichen medizinischen Gerätes, insbesondere eines implantierbaren medizinischen Gerätes wie ein Herzschrittmacher, Defibrillator oder dergleichen, mittels einer Anordnung gemäß einem der Ansprüche 1 bis 7, mit den Verfahrensschritten:
- Auswählen eines zu programmierenden persönlichen Gerätes,
- Zusammenstellen eines Programmierauftrags für das ausgewählte persönliche Gerät,
- Bestimmen mindestens eines Programmierschlusszeitpunktes für den Programmierauftrag, bis zu dem ein Übertragungsschritt des Programmierauftrags und/oder ein Ausführen des Programmierauftrags abgeschlossen sein sollen,
- Überwachen eines jeweiligen Programmierschlusszeitpunktes durch Empfangen und Auswerten von entsprechenden Programmierbestätigungen, und
- automatisches Löschen oder Stornieren eines Programmierauftrags, falls eine zu einem zugehörigen Programmierschlusszeitpunkt gehörende Programmierbestätigung nicht bis zu diesem Programmierschlusszeitpunkt empfangen wird.
